# FASCICULE DE BREVET EUROPEEN

(11) **EP 1 091 959 B1**
(45) Date de publication et mention de la délivrance du brevet: **23.10.2002**
(21) Numéro de dépôt: 00922769.5
(22) Date de dépôt: 27.04.2000
(51) Int. Cl.: C07D 471/04, C07D 471/14, A61K 31/437, A61K 31/4375, A61P 25/00

(54) **NOUVEAUX DERIVES DE PYRROLO-(3,4-B)QUINOLEINE, LEUR PROCEDE DE PREPARATION ET LEUR UTILISATION A TITRE DE MEDICAMENT**
PYRROLO[3,4-B]CHINOLINDERIVATE, VERFAHREN ZU IHRER HERSTELLUNG UND DIESE ENTHALTENDE ARZNEIMITTEL
NOVEL PYRROLO-(3,4-B)QUINOLINE DERIVATIVES, METHOD FOR THE PRODUCTION AND USE THEREOF AS A MEDICAMENT

(30) Priorité: 27.04.1999 FR 9905302
(43) Date de publication de la demande: 18.04.2001
(73) Titulaire: MACEF, 33000 Bordeaux (FR); LABORATOIRES BESINS INTERNATIONAL, 75003 Paris (FR)
(72) Inventeur: FOURTILLAN, Jean-Bernard, F-86000 Poitiers (FR); FOURTILLAN, Marianne, F-86000 Poitiers (FR); JACQUESY, Jean-Claude, F-86180 Buxerolles (FR); KARAM, Omar, F-86000 Poitiers (FR); ZUNINO, Fabien, F-86180 Buxerolles (FR); JOUANNETAUD, Marie-Paule, F-86000 Poitiers (FR)
(74) Mandataire: Ahner, Francis
(86) Numéro de dépôt international: FR0001123
(87) Numéro de publication internationale: WO00064897

(56) Documents cités:
- WO-A-96/08490

## Description

La présente invention concerne de nouveaux dérivés de pyrrolo[3,4-b]quinoléine de formule générale I : 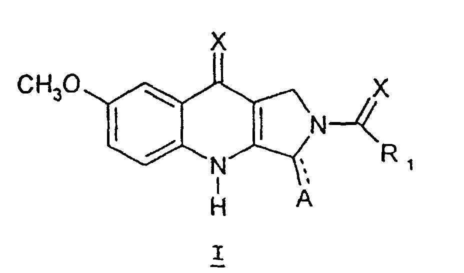 dans laquelle
X représente un atome d'oxygène ou un atome de soufre. 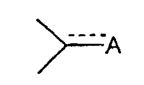 représente un groupe de formule : 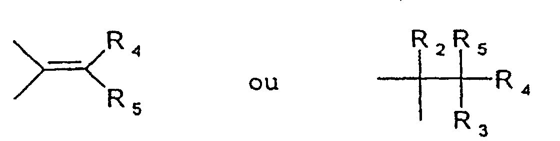
R₁ représente un radical alkyle inférieur contenant de un à trois atomes de carbone, un radical cycloalkyle inférieur contenant de trois à six atomes de carbone ou R₁ représente un radical alkyle inférieur lié à R₄ pour former un cycle à six atomes contenant 0,1 ou 2 insaturations.
R₂, R₃, R₄ et R₅ représentent indépendamment l'un de l'autre un atome d'hydrogène, un groupe alkyle inférieur contenant de un à trois atomes de carbone, un groupe cycloalkyle inférieur contenant de trois à six atomes de carbone ou un groupe phényle, les racémiques correspondants, ainsi que leurs énantiomères purs ou leurs mélanges en toutes proportions et leurs sels pharmaceutiquement acceptables.

Par alkyle inférieur, on entend des restes alkyles linéaires ou ramifiés en C₁-C₃, plus particulièrement choisis parmi les groupes méthyle, éthyle, n-propyle, iso-propyle, cyclopropyle.

Lorsque les dérivés comprennent au moins 1 carbone asymétrique, la présente invention concerne les racémiques correspondants, ainsi que leurs énantiomères purs ou leurs mélanges en toutes proportions.

Les sels thérapeutiquement acceptables des dérivés selon l'invention sont des sels usuels de la technique, organiques ou inorganiques, les chlorhydrates, les tosylates, les mesylates et les citrates, ainsi que les solvates comme les hydrates ou hémihydrates des composés de formule générale I.

La présente invention concerne plus particulièrement les dérivés de formule générale I pour laquelle de préférence X représente un atome d'oxygène ou un atome de soufre.

D'une manière préférentielle, R₁ représente avantageusement un radical alkyle inférieur, éventuellement lié à R₄ pour former un cycle à six atomes, un groupe méthyle, éthyle, n-propyle, iso-propyle, cyclopropyle.

Différents dérivés de pyrroloquinoleine ont été décrits dans l'état de la technique à titre d'intermédiaires de synthèse par :

I. Pendrak, R. Winttrock, W.D. Kingsbury, J.Org.Chem, Vol 60, N°9, 1995, Page 2912-2915. T.Sugasowa, T. Toyoda, K. Saskura, Tetrahedron.Lett, N°50, Page 5109-5112; J.M.D Fortunak, A.R. Mastrocola, M. Mellinger, J.L. Wood, Tetrahedron. Lett; Vol 35, N°32, Page 5.763-5764; D.P. Curran, H. Liu, H. Josien, Tetrahedron Lett, Vol 52, N°35, page 11385-11404; J.M.D Fortunak, A.R. Mastrocola, M. Mellinger, N.J. Sisti, J.L. Wood,Z.P. Zhung Tetrahedron. Lett; Vol 37, N°32, Page 5683-5686; D.L. Comins, J.K. Saha, J. Org. Chem, Vol 61, N°26, page 9623-9624; A.I. Meyers et al, J. Org. Chem,Vol 38, N°8, 1993; S. Daneshefsky, R. Volkmann, S.B. horwitz, Tetrahedron Lett, N°27, 1973, Page 2521, 2524; G. Stork, A.G. Schultz, J. Am. Chem. Soc, Vol 93, N°16, 1971; J. H. Rigby, D.M. Danca Tetrahedron Lett, Vol 38, N°28, 1997, Page 4969, 4972; M. Kitajima, S. Mosomoto, H. Takayama, N. Aimi, Tetrahedron. Lett, Vol 38, N°24, 1997, Page 4255-4258; M.Boch, T. Korth, J.M. Nelke, D. pike, H. Radunz, Chem. Ber, 105, 1992, Page 2126-2142; M. Naoko, S. Takumchi, K. Yoshinori, S. Tako, Synlett, 1997, Page 298-300.; J.M.D Fortunak, A.R. Mastrocola, M. Mellinger, N.J. Sisti, J.L. Wood, Z.P. Zhung Tetrahedron. Lett; Vol 37, N°32, Page 5679-5682; I. Pendrak, R. Winttrock, W.D. Kingsbury, J.Org.Chem, Vol 60, N°9, 1995, Page 2912-2915; J. Warneke, E. Winterfeldt, Chem. Ber, 105 1972, Page 2120-2125.

T.Yaegashi et Al (CA. 157128,960307); S.Sawada et Al (EP 296612,881228); B.R. Vishnuvajjala et Al (US 4943579,900724); H. Akimoto et Al (EP 556585,930825); E. Bombardelli et Al (EP 685401, 951206) E. bombardelli, L. Verotta (PCT INT Appl W.O 97 43, 290) ont décrit des dérivés de pyrroloquinoleine qui présentent une activité thérapeutique, cependant cette activité n'est pas une activité hypnotique ou sédative.

La présente invention concerne les dérivés de pyrrolo[3,4-b]quinoléine de formule générale I tels que définis ci-dessus.

Les dérivés de formule générale I, pour laquelle X représente un atome d'oxygène, peuvent être obtenus directement par oxydation des composés de formule générale II 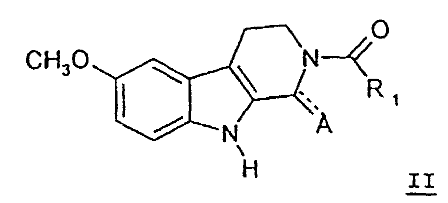 pour laquelle R₁ et A sont définis ci-dessus, avec de l'oxygène en présence d'une base telle que l'hydrure de sodium ou le tertiobutylate de potassium, ou avec un periodate.

Les analogues soufrés de formule générale I ( X = S), sont obtenus à partir des dérivés oxygénés de formule I ( X = O), correspondants par l'action du réactif de Lawesson, ou par action de pentasulfure de phosphore.

Les exemples ci-après de préparations de dérivés selon l'invention permettent d'illustrer la présente invention.

Des exemples de dérivés de formule générale I, pour lesquels :
A) X représente un atome d'oxygène
et 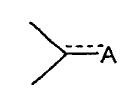 représente un groupe de formule 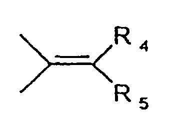
- lorsque R₄ et R₅ représentent chacun un atome d'hydrogène
   sont reportés dans le Tableau I ci après 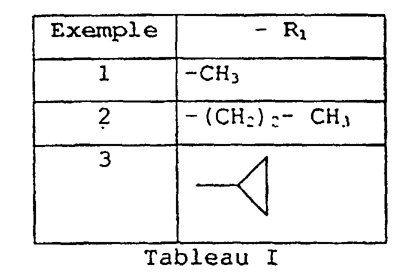
- lorsque R₁ et R₄ sont liés pour former un cycle correspondant à la formule III 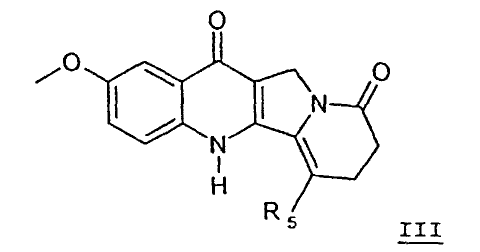 sont reportés dans le tableau II ci-après

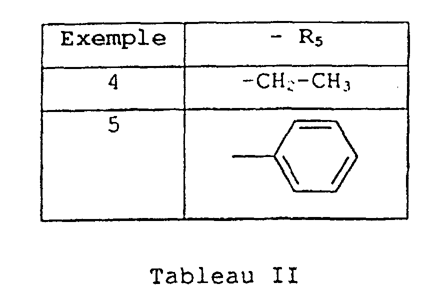

B) X représente un atome d'oxygène ou de soufre, et 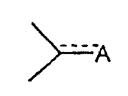 Représente un radical divalent de formule 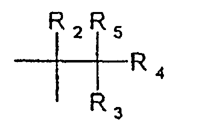
- lorsque R₃, R₄ et R₅ représentent chacun un atome d'hydrogène et R₁ représente un méthyle
sont reportés dans le Tableau III ci après

**Tableau III**

| | | |
|---|---|---|
| Exemple | X | -R₂ |
| 6 | O | -H |
| 7 | S | -H |
| 8 | O | -CH₃ |

- lorsque R₁ et R₄ sont liés pour former un cycle correspondant à la formule IV, R₂ représente un atome d'hydrogène, et X représente un atome d'oxygène

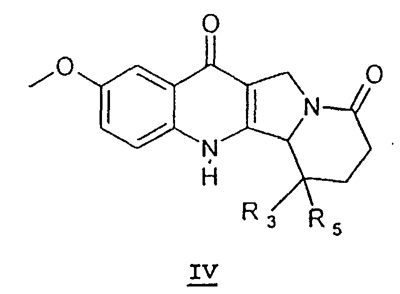

Sont reportés dans le tableau IV ci-après

**Tableau IV**

| | | |
|---|---|---|
| Exemple | R₃ | -R₅ |
| 9 | H | -CH₂-CH₃ |
| 10 | -CH₂-CH₃ | -CH₂-CH₃ |

### EXEMPLE 1

Formule :C₁₅H₁₄N₂O₃ M = 270,28 g.mol⁻¹

### Structure :

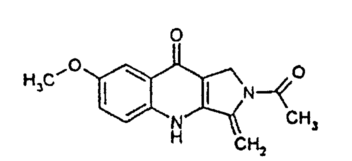

### 2-acétyl-7-méthoxy-3-méthylène-9-oxo-1,3,4,9-tetrahydropyrrolo[3,4-b]quinoléine

### Préparation :

A une solution de 1-méthylène-2-acétyl-6-méthoxy-1,2,3,4-tétrahydro-β-carboline (1g - 3,9 mmol) dans du DMF (5 ml) on additionne le NaH (1,5éq - suspension dans l'huile à 60%). Le mélange est agité sous atmosphère d'oxygène pendant une nuit. Ensuite le DMF est distillé sous pression réduite. Le brut est lavé successivement avec de l'acétate d'éthyle, du méthanol puis de l'éther. Après séchage on obtient 2-acétyl-7-méthoxy-3-méthylène-9-oxo-1,3,4,9-tetrahydro-pyrrolo[3,4-b]quinoléine.

**RMN** ^{**1**}**H** : MeOH-d₄ : 2,30 (s, 3H, CH₃CO-Nb) ; 3,89 (s, 3H, CH₃O) ; 4,87 (s, 2H, CH₂-Nb) ; 5,69 (s, 1H, H-vinylique) ; 6,03 (s, 1H, H-vinylique) ; 7,14 (dd, 1H, J₁=9Hz, J₂=3Hz, H-6) ; 7,68 (m, 2H, H-5 et H-8) ; 8,53 (s large, 1H, NH).
**Spectre de Masse** :
m/z : 270 (M⁺⁻), 228 (100), 213, 199, 184, 155

### EXEMPLE 2

Formule : C₁₇H₁₈N₂O₃ M = 298,34g.mol⁻¹

### Structure :

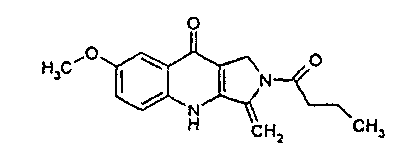

### 2-butyryl-7-méthoxy-3-méthylène-9-oxo-1,3,4,9-tetrahydropyrrolo[3,4-b]quinoléine

### Préparation :

A une solution de 1-méthylène-2-butyryl-6-méthoxy-1,2,3,4-tétrahydro-β-carboline (160 mg - 0,6 mmol) dans du DMF (3 ml) on additionne le NaH (1,5éq - suspension dans l'huile à 60%). Le mélange est agité sous atmosphère d'oxygène pendant 6 heures. Ensuite le DMF est distillé sous pression réduite. Le brut est lavé plusieurs fois avec de l'acétate d'éthyle. Après filtration et séchage on obtient 2-butyryl-7-méthoxy-3-méthylène-9-oxo-1,3,4,9-tetrahydro-pyrrolo[3,4-b]quinoléine (R^{dt}=40%).

**RMN** ^{**1**}**H** : MeOH-d₄ : 0,9 et 1,0 (2t, 3H, J=7,2 Hz, CH₃) ; 1,6 et 1,8 (2q, 2H, J=7,2Hz, CH₂CH₃) ; 2,1 et 2,5 (2t, 2H, J=7,2Hz, COCH₂CH₂) ; 3,89(s, 3H, OCH₃) ; 4,87(s, 2H, H-1) ; 5,68 (s, 1H, H-vinylique) ; 6,07 (s, 1H, H-vinylique) ; 7,14 (dd, 1H, J₁=9Hz, J₂=3Hz, H-6) ; 7,42 (d, 1H, J=3Hz, H-8) ; 7,68(d, J=9Hz, H-5) ; 8,52 (s large, 1H, NH).

**Spectre de Masse** :
m/z: 298 (M⁺⁻), 228 (100), 213, 199, 184

### EXEMPLE 3

Formule : C₁₇H₁₆N₂O₃ M = 296,32.mol⁻¹

### Structure :

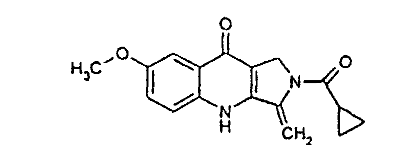

### 2-cyclopropylcarbonyl-7-méthoxy-3-méthylène-9-oxo-1,3,4,9-tetrahydro-pyrrolo[3,4-b]quinoléine

### Préparation :

A une solution de 1-méthylène-2-cyclopropylcarbonyl-6-méthoxy-1,2,3,4-tétrahydro-β-carboline (500 mg - 1,7 mmol) dans du DMF (5 ml) on additionne le NaH (1,2éq - suspension dans l'huile à 60%). Le mélange est agité sous atmosphère d'oxygène pendant 48 heures puis on rajoute à nouveau du NaH (1,1éq) et on agite une nuit. Ensuite le DMF est distillé sous pression réduite. Le brut est repris avec de l'eau puis filtré. Une solution de NH₄Cl saturée est ajoutée à la phase aqueuse. Le mélange est agité 30 minutes. Le précipité est filtré puis lavé successivement avec de l'eau, un mélange MeOH/CH₂Cl₂ (10/90), de l'acétone puis de l'acétate d'éthyle. Après séchage on obtient 2-cyclopropylcarbonyl-7-méthoxy-3-méthylène-9-oxo-1,3,4,9-tetrahydro-pyrrolo[3,4-b]quinoléine (R^{dt} = 38%).
**RMN** ^{**1**}**H :** CDCl₃ : 0,97 (m, 2H, CH₂ cyclopropyle) ; 1,1 (m, 2H, CH₂ cyclopropyle) ; 1,91(m, H, CH cyclopropyle) ; 3,90(s, 3H, OCH₃) ; 5,03(s, 2H, H-1) ; 5,43 (s, 1H, H-vinylique) ; 6,19 (s, 1H, H-vinylique) ; 7,30 (d, J=9Hz, H-8) ; 7,50 (dd, J₁=9Hz, J₂=3Hz, H-7) ; 7,67(d, J=3Hz, H-5)
**Spectre de Masse :**
m/z : 296 (M⁺⁻), 228 (100), 213, 199, 184

### EXEMPLE 4

Formule : C₁₈H₁₈N₂O₃ M = 310,35.mol⁻¹

### Structure :

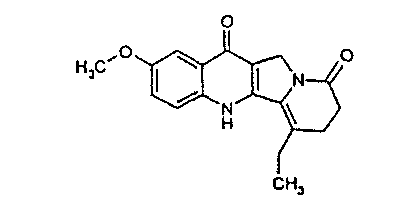

### 1-éthyl-9-méthoxy-2,3,4,6,7,12-hexahydroindolizino[1,2-b]quinoléine-4,7-dione

### Préparation :

Dans un ballon de 100 mL, on dissout 9-méthoxy-1-éthyl-2,3,4,6,7,12-hexahydroindolo [2,3-a] quinolizin-4-one (500 mg - 1,7 mmol) dans du diméthylformamide (DMF) (45 mL), on ajoute le tertiobutylate de potassium (700 mg - 6,2 mmol). Le mélange est agité sous atmosphère d'oxygène à température ambiante pendant 48 heures. Ensuite on ajoute successivement de l'eau (55 mL) et de l'acide chlorhydrique concentré (15 mL) sous agitation. Le produit précipite, après recristallisation dans un mélange éthanol-chloroforme le 1-éthyl-9-méthoxy-2,3,4,6,7,12-hexahydroindolizino[1,2-*b*]quinoléine-4,7-dione (140 mg - R=26 %)

**RMN**^{**1**}**H :** (CDCl₃/CD₃OD - 70/30):1,23 (t, 3H, CH₃) ; 2,66 (m, 4H, 2CH₂) ; 2,84(q, 4H, CH₂) 3,97(s, 3H, , CH₃O) ; 4,85 (s, 2H, CH₂) ; 7,47(d, 1H, Har) ; 7,67(s; 1H, Har), 8,17 (d, 1H, Har)
**Spectre de masse :**
m/z : 310( M⁺⁻ 100), 295, 281, 267

### EXEMPLE 5

Formule : C₂₃H₁₉NO₃ M = 357,40 g.mol⁻¹

### Structure :

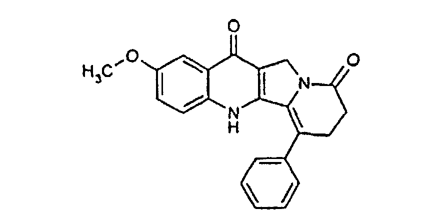

### 9-méthoxy-1-phényl-2,3,4,6,7,12-hexahydroindolizino[1,2-b]quinoléine-4,7-dione

### Préparation :

A une solution de 9-méthoxy-1-phényl-2,3,4,6,7,12-hexahydroindolo [2,3-a] quinolizin-4-one (1,45g - 40 mmol) dans du DMF (100 ml) on additionne le tertiobutylate de potassium (1,75g - 15 mmol). Le mélange est agité sous atmosphère d'oxygène pendant une nuit. Après évaporation et purification sur colonne de silice (éluant chloroforme/méthanol- 95/5). Après séchage on obtient le 9-méthoxy-1-phényl-2,3,4,6,7,12-hexahydroindolizino[1,2-*b*]quinoléine-4,7-dione (200 mg - 13 %)

**RMH** ^{**1**}**H :** CDCl₃-MeOH : 2,70 (d, j = 7.1 Hz, 2H); 2.85 (s, 1H), 3,81 (s, 3H, CH₃O); 4,77 (s, 2H) ;6,70 (d, j = 9 Hz; 1H), 7.08 (d, j = 8,7 Hz), 7.41(m, 6H).

**Spectre de Masse :**
m/z : 358 (M⁺⁻) (100), 329, 253

### EXEMPLE 6

Formule : C₁₅H₁₆N₂O₃ M = 272,30 g.mol⁻¹

### Structure :

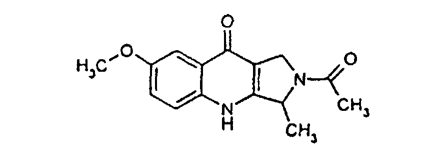

### 2-acétyl-7-méthoxy-3-méthyl-9-oxo-1,3,4,9-tetrahydropyrrolo[3,4-b]quinoléine

### Préparation :

A une solution de 1-méthyl-2-acétyl-6-méthoxy-1,2,3,4-tétrahydro-β-carboline (1,56g - 6,0 mmol) dans du DMF (20 ml) on additionne le NaH (1,2éq - suspension dans l'huile à 60%). Le mélange est agité sous atmosphère d'oxygène pendant 48 heures puis on rajoute à nouveau du NaH (1,1éq) et on agite une nuit. Ensuite le DMF est distillé sous pression réduite. Le brut est repris avec de l'eau puis filtré. Une solution de NH₄Cl saturée est ajoutée à la phase aqueuse. Le mélange est agité 30 minutes. Le précipité est filtré puis lavé successivement avec de l'eau, un mélange MeOH/CH₂Cl (10/90), de l'acétone puis de l'acétate d'éthyle. Après séchage on obtient 2-acétyl-7-méthoxy-3-méthyl-9-oxo-1,3,4,9-tetrahydro-pyrrolo[3,4-b]quinoléine (R^{dt} = 45%).

**RMN** ^{**1**}**H :** CDCl₃/MeOH-d₄ (9/1) : 1,54 (d, 3H, J=6,3Hz, CH₃) ; 2,17(s, 3H, CH₃CO-Nb) ; 3,90 (s, 3H, CH₃O) ; 4,73 (s, 2H, CH₂-Nb) ; 5,20 (q, 1H, J=6,4Hz, H-3); 7,24 (dd, 1H, J₁ = 9 Hz, J₂ = 3 Hz, H-6) ; 7,36(d, 1H, J=9Hz, H-5) ; 7,70 (d, 1H, J=3Hz, H-8)
**Spectre de Masse** :
m/z : 272 (M⁺⁻), 229 (100), 215, 199

### EXEMPLE 7

Formule : C₁₅H₁₆N₂OS₂ M = 304, 42.mol⁻¹

### Structure :

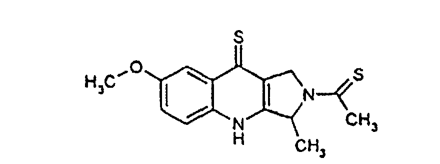

### 7-méthoxy-3-méthyl-2-thioacétyl-9-thioxo-1,3,4,9-tétrahydropyrrolo[3,4-b]quinoléine

### Préparation :

La 2-acétyl-7-méthoxy-3-méthyl-9-oxo-1,3,4,9-tetrahydropyrrolo[3,4-b]quinoléine (exemple 7) (100 mg - 0,4mmol) est mise en solution dans du toluène (15 mL) puis le réactif de Lawesson (180 mg 0,44 mmol) est ajouté avant de chauffer à reflux du toluène pendant une nuit. Après évaporation du toluène et purification sur plaque de silice (chloroforme/méthanol - 98,5/1,5) on obtient le 7-méthoxy-3-méthyl-2-thioacétyl-9-thioxo-1,3,4,9-tétrahydropyrrolo [3,4-b]quinoléine (20 mg - R=18%)

**RMN**^{**1**}**H** : (CDCl₃/CD₃OD - 95/5) : 1,70 (d, 3H, CH₃) ; 2,69(s, 3H, CH₃CS) ; 3,94(s, 3H, , CH₃O) ; 4,82 (dd, 2H, CH₂) ; 5,75(q, 1H, CH) ; 7,29(dd; 1H, Har), 7,42(d; 1H, Har), 8,23 (d, 1H, Har)
**Spectre de masse :**
m/z : 314 ( M⁺⁻ 100), 271, 245

### EXEMPLE 8

Formule : C₁₆H₁₈N₂O₃ M = 286,33 g.mol⁻¹

### Structure :

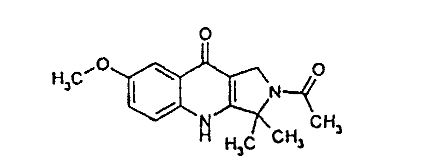

### 2-acétyl-3,3-diméthyl-7-méthoxy-9-oxo-1,3,4,9-tetrahydropyrrolo[3,4-b]quinoléine

### Préparation :

A une solution de 2-acétyl-1,1-diméthyl-6-méthoxy-1,2,3,4-tétrahydro-β-carboline (164 mg - 0.60 mmol) dans du DMF (15 mL) on additionne le tertiobutylate de potassium (269 mg - 2,39 mmol). Le mélange est agité sous atmosphère d'oxygène pendant une nuit. Ensuite un mélange acétate d'éthyle/méthanol - 1/1 (10 mL) est ajouté. Après évaporation du solvant et lavage du solide le : 2-acétyl-3,3-diméthyl-7-méthoxy-9-oxo-1,3,4,9-tetrahydro-pyrrolo[3,4-b]quinoléine est obtenu (38 mg - 22 %)

**RMN** ^{**1**}**H :** CDCl₃/MeOH-d₄ (9/1) : 1,87 (s, 6H) ; 2,19(s, 3H) ; 3,93 (s, 3H) ; 7,34 (dd, 1H) ; 7,59 (d, 1H) ; 7,70 (d, 1H)

**Spectre de Masse :**
m/z : 286 (M⁺⁻), 271, 243, 229 (100), 213

### EXEMPLE 9

Formule : C₁₈H₂₀N₂O₃ M = 312,36 g.mol⁻¹

### Structure :

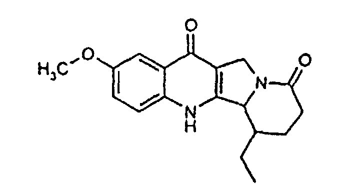

### 1-éthyl-9-méthoxy-1,2,3,4,6,7,12,12b-octahydroindolizino [1,2-b]quinoléine-4,7-dithione

### Préparation :

A une solution de 9-méthoxy-1-éthyl-1,2,3,4,6,7,12,12b-octahydroindolo[2,3-a]quinolizin-4-one (1,0g - 3,35 mmol) dans du DMF (90 ml) on additionne le tertiobutylate de potassium (1,4 g mg - 12,4 mmol). Le mélange est agité sous atmosphère d'oxygène pendant une nuit. Ensuite un mélange acétate d'éthyle/ méthanol - 1/1 (10 mL) est ajouté. Après évaporation et purification sur colonne de silice (éluant chloroforme/méthanol - 95/5) le 1-éthyl-9-méthoxy-1,2,3,4,6,7,12,12b-octahydroindolizino[1,2-*b*]quinoléine-4,7-dithione est obtenu (360 mg - 34 %)

**RMN** ^{**1**}**H :** CDCl₃/MeOH-d₄ (9/1) : 0,89(t, 3H, J=7,4Hz) ;1,10(q, 2H, J =7,4Hz), 2,07(m, 2H, ) ; 2,44(m, 3H), 3,90 (s, 3H, CH₃O) ; 4,45 (d, 1H, J = 14,8Hz) ; 4,90 (d, 1H, J=14,8Hz) ;4,99 (s, 1H), 7,26 (dd, 1H, J₁ = 9 Hz, J₂ = 2,7 Hz) ; 7,49(d, 1H, J=9Hz, H-5) ; 7,69 (d, 1H, J=2,7Hz)

**Spectre de Masse :**
m/z : 312 (M⁺⁻) (100), 283, 214, 199, 171

### EXEMPLE 10

Formule : C₂₀H₂₄N₂O₃ M = 340,43 g.mol⁻¹

### Structure :

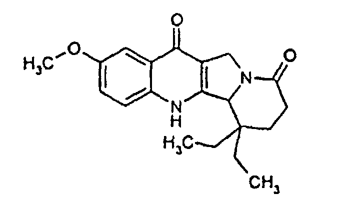

### 1, 1-diéthyl-9-méthoxy-1,2,3,4,6,7,12,12b-octahydroindolizino [1,2-b]quinoléine-4,7-dithione

### Préparation :

A une solution de 9-méthoxy-1,1-diéthyl-1,2,3,4,6,7,12,12b-octahydroindolo[2,3-a]quinolizin-4-one (80 mg - 0,24 mmol) dans du DMF (7 ml) on additionne le le tertiobutylate de potassium (113 mg - 1,0 mmol). Le mélange est agité sous atmosphère d'oxygène pendant une nuit. Ensuite un mélange acétate d'éthyle/ méthanol - 1/1 (10 mL) est ajouté. Après évaporation et purification sur colonne de silice (éluant chloroforme/méthanol - 97,5/4,5) le 1,1-diéthyl-9-méthoxy-1,2,3,4,6,7,12,12b-octahydroindolizino[1,2-*b*]quinoléine-4,7-dithione est obtenu (31 mg - 37 %)

**RMN** ^{**1**}**H :** CDCl₃/MeOH-d₄ (9/1)
**Spectre de Masse :**
m/z : 340 (M⁺⁻) 309; 214(100), 199, 171

### ACTIVITE PHARMACOLOGIQUE

### 1. ACTIVITE HYPNOTIQUE CHEZ LE CHIEN BEAGLE (mesure des états de veille et de sommeil)

Lors de chaque test, les chiens sont portés en salle d'enregistrement où ils sont connectés à un polygraphe SCHWARZER ED 24 piloté par BRAINLAB® pour WINDOWS® au moyen d'un câble souple. Le chien est ensuite placé en cage métallique de 0,70 x 1 x 0,8 m où il séjourne 150 minutes.

Les produits d'essai et le placebo ont été administrés par voie intraveineuse en bolus unique à la veine médiane antérieure. Les enregistrements sont notés par époques de 30 secondes suivant les cinq états de vigilance définis par SHELTON et coll :
- veille,
- somnolence,
- sommeil lent léger,
- sommeil lent profond
- et sommeil paradoxal.
à l'examen de l'amplitude et de la fréquence des 2 tracés frontaux d'EEG (frontal), des EOG (bilatéral) et de l'EMG (muscles de la nuque) comme suit :
- Veille : Les chiens peuvent être debout, assis ou couchés, ou mobiles entre ces positions. Ils ont les yeux ouverts. Cet état inclut tous les épisodes à voltage et fréquences multiples avec une activité musculaire.
- Somnolence : Les chiens sont couchés les yeux clos pendant la majorité du temps. Les tracés EEG présentent des cycles d'ondes lentes (5-7 Hz) sans apparition de fuseau. Des ondes lentes (4-7 Hz) synchrones et de grande amplitude apparaissent sur fond d'activité rapide. L'EMG tend à être diminué par rapport à la veille.
- Sommeil léger (SL1 ou S1) : Les chiens sont couchés en position ventrale allongée ou le plus souvent en cercle incomplet. La relaxation est totale. Les tracés EEG sont caractérisés par des complexes K et/ou des fuseaux. L'amplitude augmente.
- Sommeil profond (SL2 ou S2) : Les chiens sont couchés, myorelachés. Ils restent sans réaction à des stimulations modérées (sonores). Les ondes lentes delta (< 4 Hz) d'amplitude 70 µV au moins, constituent au moins 20 % d'une période de 30 secondes.
- Sommeil paradoxal (SP) : Les chiens sont couchés les yeux fermés et présentent parfois des contractions rapides des paupières et/ou des clonies musculaires, des muscles releveurs labiaux en particulier. Les tracés EEG sont de faible voltage et de fréquences variées. Les EOG montrent des contractions rapides. L'EMG est de faible amplitude, à l'exception des clonies de courte durée. Une période n'est classée en sommeil paradoxal que dans la mesure où celle qui précède est une période de sommeil. La fin d'une période de sommeil paradoxal est déterminée par l'apparition de complexes K ou de fuseaux, ou par l'apparition d'une activité EMG.

Les composés à tester sont administrés par voie intraveineuse au moyen d'un véhicule de composition : PEG 400/eau pour préparations injectables 50/50 (V/V) en un bolus unique de 10 ml pour 12 kg de poids vif.

Le placebo est constitué par l'administration intraveineuse du véhicule seul de composition : PEG 400/eau pour préparations injectables 50/50 (V/V) en un bolus unique de 10 ml pour 12 kg de poids vif.

La répartition du temps d'observation passé à chacun des stades de vigilance et de sommeil ainsi que les temps de latence d'apparition des premiers stades de chaque type de sommeil sont rapportés au Tableau I.

Les composés des exemples 4 et 5 ont un pouvoir hypnotique net. Ils induisent un sommeil caractérisé par une proportion élevée d'ondes lentes. La durée des périodes vigiles est diminuée et celle des phases non vigiles augmentées pour chacun de ces deux composés. Les temps de latence d'apparition de la somnolence, du sommeil lent léger et profond sont également diminués.

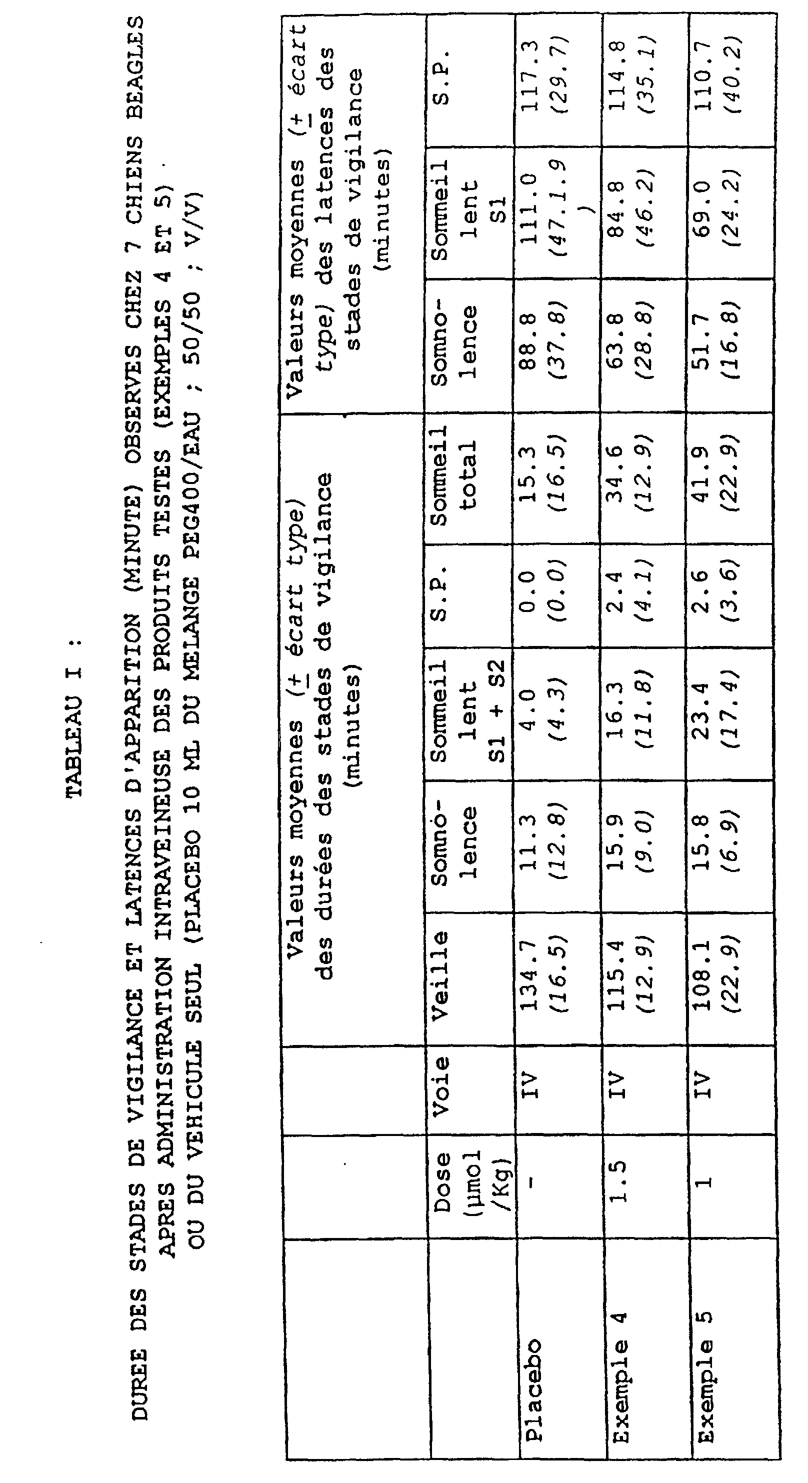

### 2. ACTIVITES HYPNOTIQUES ET SEDATIVES CHEZ LE POUSSIN

Les effets hypnotiques et sédatifs des dérivés, selon l'invention, préparés ci-dessus (dont les résultats des tests sont indiqués dans le Tableau II ci-après), ont été comparés à ceux de 3 produits de référence, le diazépam, le pentobarbital sodique, la mélatonine ainsi qu'à 2 composés psychostimulants à propriétés hallucinogènes : le 10-méthoxy harmalan et l'harmaline qui sont des 3,4-dihydro-β-carbolines, chez des poussins de souche chair label JA657, âgés de 10 à 14 jours. Les animaux sont soumis à des programmes d'éclairement alterné comportant 12 h d'obscurité (20 h à 8 h) et 12 h d'éclairement (8 h à 20 h). La température d'ambiance est de 25°C pendant la première semaine d'élevage des poussins et de 22°C à partir de la deuxième semaine. Pendant la journée, l'éclairement est assuré par une lampe halogène (300 W), placée à 30 cm au-dessus du plancher du vivarium. Pendant les tests, les poids vifs des poussins ont varié entre 85 et 120 g. Les tests sont réalisés entre 14 et 15 h. Les poussins sont allotés par groupes de 3, dans des vivariums identiques de 30 cm x 50 cm x 30 cm. Les produits testés sont administrés par voie intramusculaire (IM) dans le muscle pectoral majeur, en solution dans un mélange Ethanol/PEG400/Eau distillée (25/50/25, V/V/V), à raison de 0,2 ml de solution pour 100 g de poids vif. Les doses administrées pour les produits testés (nouveaux composés de l'invention et substances de référence) varient de 0,5 µmoles à 2 µmoles pour 100 g de poids vif. Le placebo correspond à 0,2 ml du mélange Ethanol/PEG400/Eau distillée (25/50125, V/V/V) pour 100 g de poids vif. L'éthanol étant utilisé comme solvant, son effet a été comparé préalablement à celui du soluté physiologique (soluté NaCl à 0,9 p. 100) ou de l'eau distillée.

Les solutions des produits testés ont été préparées extemporanément par dilution successive d'une solution mère, obtenue .à partir de 5 à 20 µmoles de produit exactement pesé, additionné de 0,5 ml d'éthanol pur, 1 ml de PEG 400, puis complétées à 2 ml avec 0,5 ml d'eau distillée pour préparation injectable. Dans le Tableau II sont présentés les résultats obtenus après administrations IM de doses comprises entre 0,5 et 2 µmoles de produits testés, en solution dans 0,2 ml du mélange Ethanol/PEG400/Eau distillée (25/50/25, V/V/V) pour 100 g de poids vif. Pour chaque poussin, le volume injecté est ajusté, en fonction du poids vif réel, à 0,2 ml pour 100 g de poids vif.

Les paramètres observés sont l'activité locomotrice et l'état de veille des poussins pendant 2 h, soit l'équivalent de 6 cycles théoriques veille-sommeil du poussin de cet âge. Ils sont enregistrés par caméra vidéo pendant 90 minutes, les 30 premières minutes étant le temps d'adaptation au dispositif.

Cinq stades de vigilance ont été définis :
- stade 1 : veille active ;
- stade 2 : animal couché, maintien de la tête avec tonicité, oeil ouvert ;
- stade 3 : sommeil léger, animal assoupi : oeil fermé avec ouverture intermittente, posture immobile non modifiée par la stimulation ;
- stade 4 : sommeil profond couché : relâchement du cou, posture caractéristique tête sous l'aile ou en arrière ;
- stade 5 : sommeil debout : oeil fermé, immobile, tête tombante (catatonique).

Ces cinq stades correspondent approximativement aux stades de vigilance et de sommeil définis à l'examen des tracés électro-encéphalographiques dans cette espèce. La correspondance est la suivante :
. sommeil profond couché : stade 4 = « slow wave sleep » (SWS)
. sommeil debout = « sleep-like state I » (SLSI).

Le stade 3, assoupi, pourrait correspondre à des phases de sommeil paradoxal, avec agitation de la tête, par exemple.

L'observation des poussins est réalisée par un observateur entraîné avec un contrôle vidéo continu pendant au moins 1 heure après le réveil des animaux.

Deux stimuli ont été utilisés pour confirmer les observations du comportement des poussins à intervalles réguliers :
- le bruit causé par le choc d'un objet en plastique sur la vitre du vivarium, comparable à celui du bec d'un poussin sur la vitre, correspond à un stimulus modéré. Il est pratiqué à chaque période d'observation (soit toutes les 5 minutes) ;
- et la présentation d'une mangeoire métallique remplie avec l'aliment habituel, laissée 2 minutes dans le vivarium. Il s'agit d'un stimulus puissant faisant appel à la vision, l'ouïe et l'odorat. Elle est pratiquée toutes les 15 minutes, c'est-à-dire 6 fois, au moins, à chaque essai.

Le réveil est défini par l'apparition du comportement élaboré conscient de recherche et consommation de nourriture ou de besoin.

Le Temps du Sommeil (TS) est défini par la somme des durées des phases de sommeil léger (stade 3), sommeil profond (stade 4) et sommeil debout (stade 5). Le Temps de Sédation, postériéur au réveil, correspond au stade 2.

Le Temps d'Assoupissement (TA) est égal (à 1 minute près) au temps nécessaire au passage d'état de veille active (stade 1) à un état non vigile (stades 3, 4 et 5).

Les effets hypnotiques et sédatifs des produits d'essai, sur l'activité diurne de poussins, âgés de 10 à 14 jours, soumis à un programme d'éclairement permanent dès la naissance pendant 48 h, puis à un programme d'éclairement alterné de 12 h de jour (8 h - 20 h) et 12 h d'obscurité (20 h - 8 h) jusqu'au jour de l'essai; sont reportés au Tableau II ci-après. Les tests sont réalisés le jour entre 14 h et 15 h.

Pour chaque produit testé, plusieurs séries de masures ont été réalisées sur des lots de 3 animaux, chaque valeur indiquée est la moyenne dans chaque lot de 3 poussins. Lorsque le nombre de lots est supérieur à 2, les chiffres indiqués sont les valeurs moyennes limites observées.

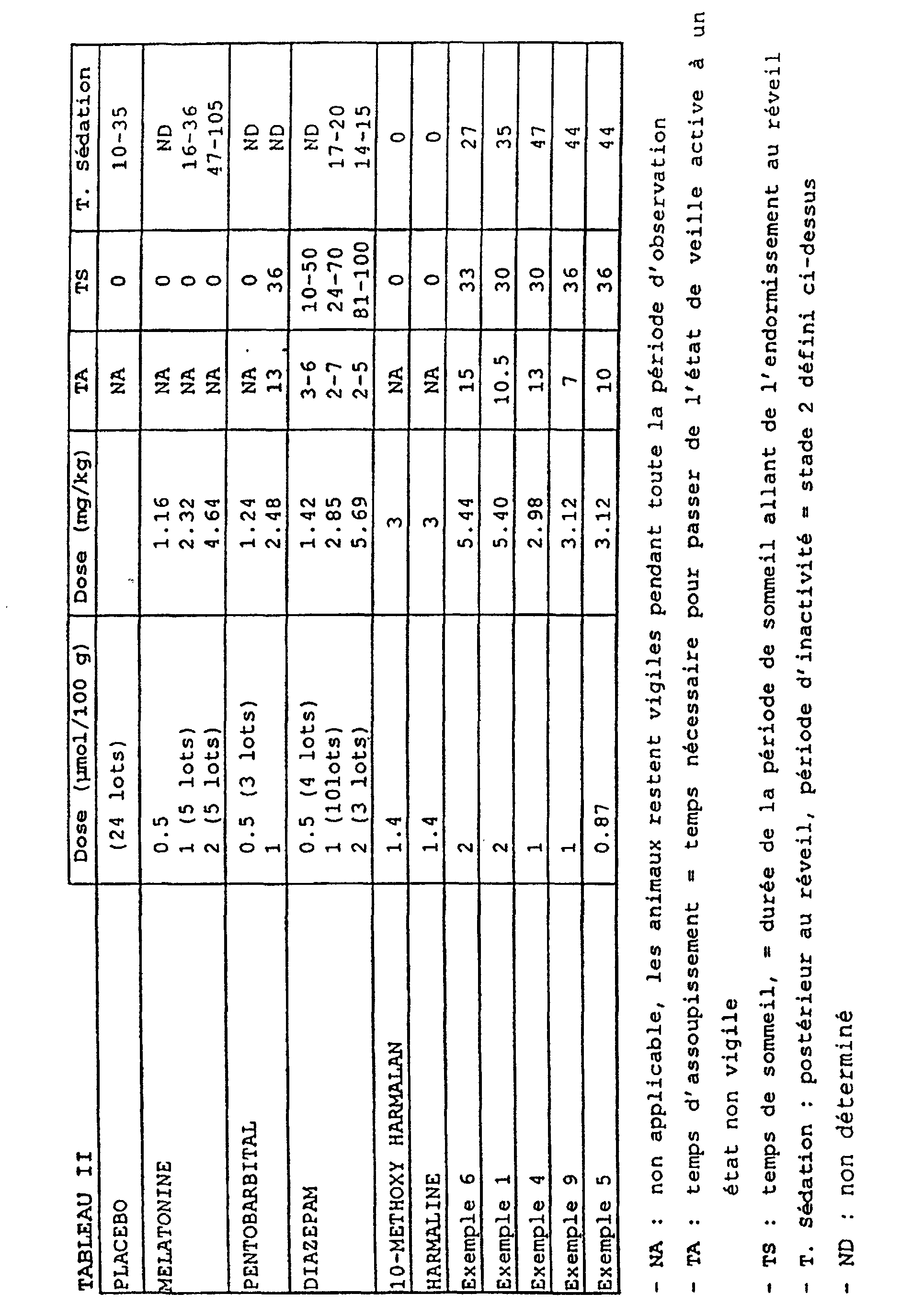

Sans vouloir être lié à aucune théorie, on peut postuler l'argumentation suivante.

Dans les conditions de réalisation du test (horaires d'administration pendant la phase d'éclairement des animaux entre 14 h et 15 h) l'activité hypnotique de la mélatonine est nulle.

En soumettant. successivement des poussins à des programmes d'éclairements alterné et permanent, nous avons démontré expérimentalement que la mélatonine n'a pas d'activité hypnotique directe, propre à sa structure. Son activité hypnotique dépend de l'activité de l'Enzyme N-Acétyl Transférase (NAT) dans la glande pinéale du poussin au moment de l'administration de la mélatonine. L'enzyme NAT est une enzyme d'acétylation. En présence de l'enzyme NAT dans la glande pinéale du poussin, l'administration IM de mélatonine induit un effet hypnotique de forte intensité (temps de sommeil compris entre 250 et 300 minutes pour une dose égale à 1 µM de mélatonine / 100 g de poids vif). La mélatonine est donc le précurseur de métabolites acétylés à activité hypnotique directe. Les composés décrits dans la présente invention sont des analogues des métabolites acétylés hypnotiques de la mélatonine.

Contrairement à la mélatonine, tous les dérivés de l'invention décrits ci-dessus ont des activités hypnotiques ou sédatives directes, qui sont indépendantes de l'heure d'administration, c'est-à-dire du taux de l'enzyme N-Acétyl Transférase dans le SNC.

Les résultats obtenus montrent, pour les dérivés selon l'invention, un effet hypnotique supérieur à celui des produits de référence (pentobarbital, mélatonine) et équivalent ou même supérieur à celui du diazépam.

Les dérivés selon l'invention sont donc des médicaments particulièrement avantageux pour le traitement de maladies Liées aux désordres de l'activité de la mélatonine. Ces dérivés peuvent notamment être utilisés en tant que médicament hypnotique ou sédatif.

## Revendications

1. Dérivés de pyrrolo[3,4-b]quinoléine de formule générale I 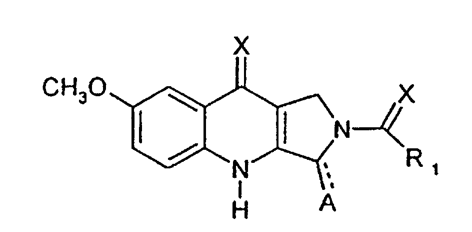 dans laquelle
X représente un atome d'oxygène ou un atome de soufre, 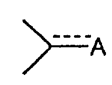 représente un radical divalent de formule 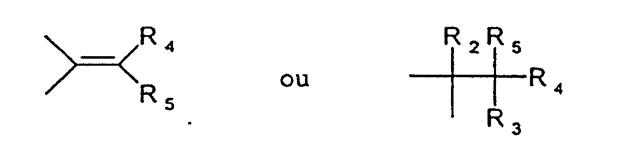
R₁ représente un radical alkyle inférieur contenant de un à trois atomes de carbone, un radical cycloalkyle inférieur contenant de trois à six atomes de carbone, ou R₁ représente un radical alkyle inférieur lié à R₄ pour former un cycle à six atomes contenant 0,1 ou 2 insaturations,
R₂, R₃, R₄ et R₅ représentent indépendamment l'un de l'autre un atome d'hydrogène, un groupe alkyle inférieur contenant de un à trois atomes de carbone, un groupe cycloalkyle inférieur contenant de trois à six atomes de carbone, ou un groupe phényle,
les racémiques correspondants, ainsi que leurs énantiomères purs ou leurs mélanges en toutes proportions et leurs sels pharmaceutiquement acceptables.

2. Dérivé selon la revendication 1 dans laquelle R₁ représente un groupe méthyle, éthyle, n-propyle, iso-propyle, cyclopropyle.

3. Dérivé selon la revendication 1 ou 2 **caractérisé en ce qu'**il est choisi dans le groupe formé par les composés suivants :
- 2-acétyl-7-méthoxy-3-méthylène-9-oxo-1,3,4,9-tetrahydro-pyrrolo[3,4-*b*]quinoléine,
- 2-butyryl-7-méthoxy-3-méthylène-9-oxo-1,3,9,9-tetrahydro-pyrrolo[3,4-*b*]quinoléine,
- 2-cyclopropylcarbonyl-7-méthoxy-3-méthylène-9-oxo-1,3,4,9-tetrahydro-pyrrolo[3,4-*b*]quinoléine,
- 1-éthyl-9-méthoxy-2, 3,4,6,7,12-hexahydroindolizino[1,2-*b*]quinoléine-4,7-dione,
- 9-méthoxy-1-phényl-2,3,4,6,7,12-hexahydroindolizino[1,2-*b*]quinoléine-4,7-dione
- 2-acétyl-7-méthoxy-3-méthyl-9-oxo-1,3,4,9-tetrahydro-pyrrolo[3,4-*b*]quinoléine,
- 7-méthoxy-3-méthyl-2-thioacétyl-9-thioxo-1,3,4,9-tétrahydropyrrolo[3,4-b]quinoléine,
- 2-acétyl-3,3-diméthyl-7-méthoxy-9-oxo-1,3,4,9-tetrahydro-pyrrolo[3,4-*b*]quinoléine,
- 1-éthyl-9-méthoxy-1,2,3,4,6,7,12,12*b*-octahydroindolizino[1,2-*b*]quinoléine-4,7-dithione,
1,1-diéthyl-9-méthoxy-1,2,3,4,6,7,12,12b-octahydroindolizino[1,2-b]quinoléine-4,7-dithione.

4. Procédé de préparation des dérivés de formule générale I selon l'une des revendications 1 à 3, pour laquelle X représente un atome d'oxygène, par oxydation des composés de formule générale II 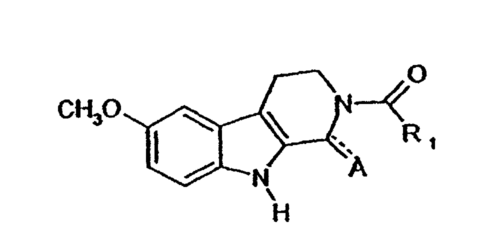 Pour laquelle R1 et A sont définis à la revendication 1, avec de l'oxygène en présence d'une base telle que l'hydrure de sodium ou le tertiobutylate de potassium, ou avec un periodate.

5. Procédé de préparation des dérivés de formule générale I selon l'une des revendications 1 à 3, pour laquelle X représente un atome de soufre (X = S) par action du réactif de Lawesson, ou du pentasulfure de phosphore sur les dérivés oxygénés de formule I (X = O), correspondants.

6. Dérivé selon l'une des revendications 1 à 3 ou obtenu par le procédé de préparation selon l'une des revendications 4 ou 5 pour son utilisation en tant que médicament.

7. Dérivé selon la revendication 6 pour son utilisation en tant que médicament hypnotique.

8. Dérivé selon la revendication 6 pour son utilisation en tant que médicament sédatif.

## Patentansprüche

1. Derivate von Pyrrolo[3,4-b]chinolin der allgemeinen Formel I 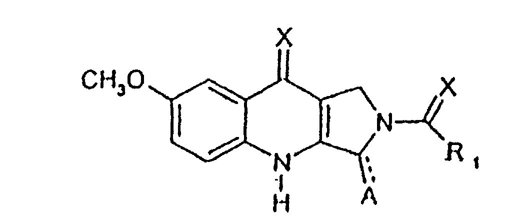 in der
X ein Sauerstoffatom oder ein Schwefelatom darstellt, 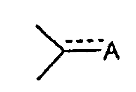 einen zweiwertigen Rest der Formel 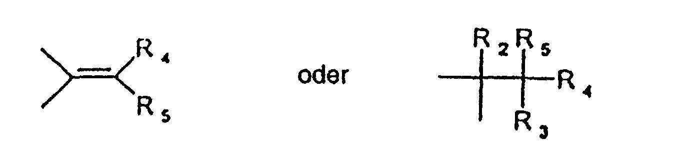 darstellt,
R₁ einen Niederalkylrest, der ein bis drei Kohlenstoffatome enthält, einen Niedercycloalkylrest darstellt, der drei bis sechs Kohlenstoffatome enthält, oder R₁ einen Niederalkylrest darstellt, der unter Bildung eines Cyclus mit sechs Atomen, der 0, 1 oder 2 Unsättigungen enthält, an R₄ gebunden ist,
R₂, R₃, R₄ und R₅ unabhängig voneinander ein Wasserstoffatom, eine Niederalkylgruppe, die ein bis drei Kohlenstoffatome enthält, eine Niedercycloalkylgruppe, die drei bis sechs Kohlenstoffatome enthält, oder eine Phenylgruppe darstellen,
die entsprechenden Racemate sowie ihre reinen Enantiomere oder ihre Mischungen in allen Verhältnissen und ihre pharmazeutisch annehmbaren Salze.

2. Derivat nach Anspruch 1, in dem R₁ eine Methyl-, Ethyl-, n-Propyl-, Isopropyl-, Cyclopropylgruppe darstellt.

3. Derivat nach Anspruch 1 oder 2, **dadurch gekennzeichnet, dass** es aus der Gruppe ausgewählt ist, die durch die folgenden Verbindungen gebildet wird:
- 2-Acetyl-7-methoxy-3-methylen-9-oxo-1,3,4,9-tetrahydropyrrolo[3,4-b]-chinolin,
- 2-Butyryl-7-methoxy-3-methylen-9-oxo-1,3,4,9-tetrahydropyrrolo[3,4-b]-chinolin,
- 2-Cyclopropylcarbonyl-7-methoxy-3-methylen-9-oxo-1,3,4,9-tetrahydropyrrolo[3,4-b]chinolin,
- 1-Ethyl-9-methoxy-2,3,4,6,7,12-hexahydroindolizino[1,2-b]chinolin-4,7-dion,
- 9-Methoxy-1-phenyl-2,3,4,6,7,12-hexahydroindolizino[1,2-b]chinolin-4,7-dion,
- 2-Acetyl-7-methoxy-3-methyl-9-oxo-1,3,4,9-tetrahydropyrrolo[3,4-b]-chinolin,
- 7-Methoxy-3-methyl-2-thioacetyl-9-thioxo-1,3,4,9-tetrahydropyrrolo[3,4-b]chinolin,
- 2-Acetyl-3,3-dimethyl-7-methoxy-9-oxo-1,3,4,9-tetrahydropyrrolo[3,4-b]-chinolin,
- 1-Ethyl-9-methoxy-1,2,3,4,6,7,12,12b-octahydroindolizino[1,2-b]chinolin-4,7-dithion,
- 1,1-Diethyl-9-methoxy-1,2,3,4,6,7,12,12b-octahydroindolizino[1,2-b]-chinolin-4,7-dithion.

4. Verfahren zur Herstellung von Derivaten der allgemeinen Formel I gemäß einem der Ansprüche 1 bis 3, in der X ein Sauerstoffatom darstellt, durch Oxidation von Verbindungen der allgemeinen Formel II 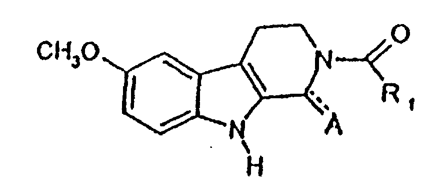 in der R₁ und A wie in Anspruch 1 definiert sind, mit Sauerstoff in Anwesenheit einer Base wie Natriumhydrid oder Kaliumtertiärbutylat oder mit einem Periodat.

5. Verfahren zur Herstellung von Derivaten der allgemeinen Formel I gemäß einem der Ansprüche 1 bis 3, in der X ein Schwefelatom darstellt (X = S), durch Einwirkung des Lawesson-Reagenz oder von Phosphorpentasulfid auf die entsprechenden Sauerstoff-Derivate der Formel I (X = O).

6. Derivat nach einem der Ansprüche 1 bis 3 oder erhalten durch das Herstellungsverfahren gemäß einem der Ansprüche 4 oder 5 für seine Verwendung als Medikament.

7. Derivat nach Anspruch 6 für seine Verwendung als Hypnotikum.

8. Derivat nach Anspruch 6 für seine Verwendung als Sedativum.

## Claims

1. Pyrrolo[3,4-b]quinoline derivatives of general formula I: 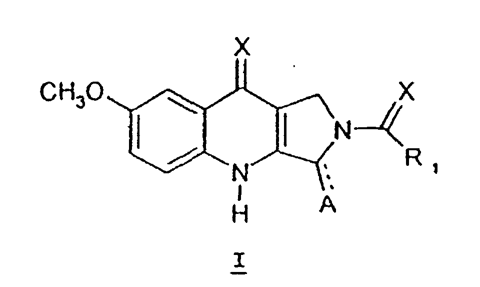 in which
X represents an oxygen atom or a sulphur atom, 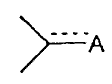 represents a divalent radical of formula: 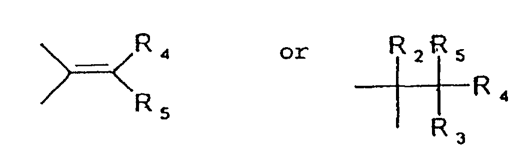
R₁ represents a lower alkyl radical containing from one to three carbon atoms, a lower cycloalkyl radical containing from three to six carbon atoms or R₁ represents a lower alkyl radical linked to R₄ to form a 6-atom ring containing 0, 1 or 2 unsaturations,
R₂, R₃, R₄ and R₅ represent, independently of each other, a hydrogen atom, a lower alkyl group containing from one to three carbon atoms, a lower cycloalkyl group containing from three to six carbon atoms or a phenyl group,
the corresponding racemic mixtures, as well as the pure enantiomers thereof or mixtures thereof in all proportions and the pharmaceutically acceptable salts thereof.

2. Derivative according to Claim 1, in which R₁ represents a methyl, ethyl, n-propyl, isopropyl or cyclopropyl group.

3. Derivative according to Claim 1 or 2, **characterized in that** it is chosen from the group formed by the following compounds:
- 2-Acetyl-7-methoxy-3-methylene-9-oxo-1,3,4,9-tetrahydropyrrolo[3,4-b]quinoline,
- 2-Butyryl-7-methoxy-3-methylene-9-oxo-1,3,4,9-tetrahydropyrrolo[3,4-b]quinoline,
- 2-Cyclopropylcarbonyl-7-methoxy-3-methylene-9-oxo-1,3,4,9-tetrahydropyrrolo[3,4-b]quinoline,
- 1-Ethyl-9-methoxy-2,3,4,6,7,12-hexahydroindolizino[1,2-b]quinoline-4,7-dione,
- 9-Methoxy-1-phenyl-2,3,4,6,7,12-hexahydroindolizino[1,2-b]quinoline-4,7-dione,
- 2-Acetyl-7-methoxy-3-methyl-9-oxo-1,3,4,9-tetrahydropyrrolo[3,4-b]quinoline,
- 7-Methoxy-3-methyl-2-thioacetyl-9-thioxo-1,3,4,9-tetrahydropyrrolo[3,4-b]quinoline,
- 2-Acetyl-3,3-dimethyl-7-methoxy-9-oxo-1,3,4,9-tetrahydropyrrolo[3,4-b]quinoline,
- 1-Ethyl-9-methoxy-1,2,3,4,6,7,12,12b-octahydroindolizino[1,2-b]quinoline-4,7-dithione,
- 1,2-Diethyl-9-methoxy-1,2,3,4,6,7,12,12b-octahydroindolizino[1,2-b]quinoline-4,7-dithione.

4. Process for preparing the derivatives of general formula I according to one of Claims 1 to 3, for which X represents an oxygen atom, by oxidation of the compounds of general formula II 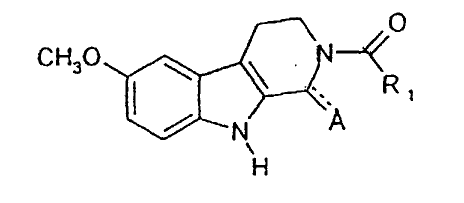 for which R₁ and A are defined in Claim 1, with oxygen in the presence of a base such as sodium hydride or potassium tert-butoxide, or with a periodate.

5. Process for preparing the derivatives of general formula I according to one of Claims 1 to 3, for which X represents a sulphur atom (X = S), by the action of Lawesson's reagent, or phosphorus pentasulphide, on the corresponding oxygen derivatives of formula I (X = O).

6. Derivative according to one of Claims 1 to 3 or obtained by the preparation process according to either of Claims 4 and 5, for its use as a medicinal product.

7. Derivative according to Claim 6, for its use as a hypnotic medicinal product.

8. Derivative according to Claim 6, for its use as a sedative medicinal product.
